# EUROPEAN PATENT APPLICATION

(11) **EP 4 454 705 A1**
(43) Date of publication of application: **30.10.2024**
(21) Application number: 23196899.1
(22) Date of filing: 12.09.2023
(51) Int. Cl.: A61P 27/02, A61K 31/50, C07K 16/28

(54) **EGFR ANTAGONISTS FOR THE TREATMENT OF DISEASES INVOLVING UNWANTED MIGRATION, PROLIFERATION, AND METAPLASIA OF RETINAL PIGMENT EPITHELIUM (RPE) CELLS**

(30) Priority: 28.04.2023 EP 23170806
(71) Applicant: Jonas, Jost, B., 69115 Heidelberg (DE); Panda-Jonas, Songhomitra, 69469 Weinheim (DE); Jonas, Shefali Brinda, 69469 Weinheim (DE); Jonas, Rahul Arvo, 69469 Weinheim (DE)
(72) Inventor: Jonas, Jost, B., 69115 Heidelberg (DE); Panda-Jonas, Songhomitra, 69469 Weinheim (DE); Jonas, Shefali Brinda, 69469 Weinheim (DE); Jonas, Rahul Arvo, 69469 Weinheim (DE)
(74) Representative: Ullrich & Naumann PartG mbB

(57) **Abstract**

The present invention relates to a pharmaceutical composition for use in the treatment of a disease involving unwanted migration, proliferation and metaplasia of retinal pigment epithelium (RPE) cells in a subject, wherein the pharmaceutical composition comprises at least one epidermal growth factor receptor (EGFR) antagonist.

## Description

### FIELD OF THE INVENTION

The present invention relates generally to the field of pharmaceutical compositions and, more specifically, to the development of pharmaceutical compositions for the treatment of diseases involving unwanted migration, proliferation, and metaplasia of retinal pigment epithelium (RPE) cells in a subject.

### BACKGROUND OF THE INVENTION

### Age-related macular degeneration (AMD)

Age-related macular degeneration (AMD) is one of the most common causes for irreversible vision impairment and blindness worldwide (Bourne et al. 2013, Flaxman et al. 2017). AMD can be differentiated into a non-exudative, dry form with degenerative changes and eventual loss of retinal pigment epithelium (RPE) cells, and into a wet, or exudative/neovascular/proliferative form (Lim et al. 2012, Mitchell et al. 2018). Changes of the RPE belong to the hallmarks of AMD (Mitchell et al. 2018; Guymer et al. 2023). These RPE alterations include an RPE cell loss in association with the development of geographic atrophy in the late stage of AMD, and RPE cell proliferations in the subretinal space between the retina and Bruch's membrane in the case of the neovascular or exudative form of AMD as well as an RPE cell migration / proliferation into the retina in the intermediate and late stage of AMD (Cao et al., 2021; Fleckenstein et al. 2018; Sadda et al. 2018; Guymer et al. 2020; Spaide et al. 2018).

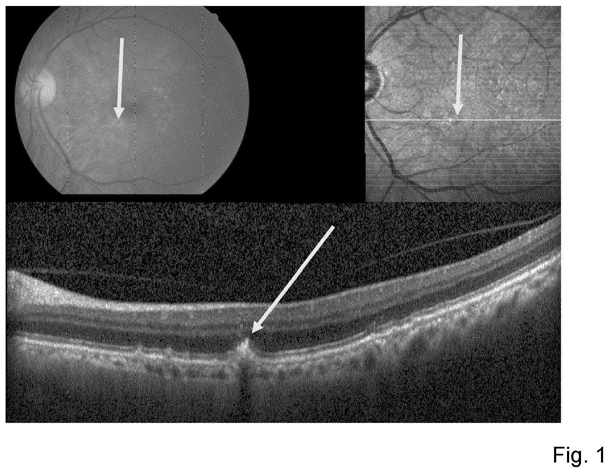

In the case of the non-exudative or intermediate stage of AMD, affected eyes can show irregular pigmentations in the macular region. It indicates a loss of the original function of these RPE cells in the macular region of eyes with AMD and a metaplastic change of the cells. Performing a combined histologic and clinical study, Cao and colleagues recently reported about the migration/proliferation of single, or few clustered, macular RPE cells into the retina in eyes without choroidal neovascularization (Cao et al. 2021). Cao and associates observed that a plume morphology of the intraretinally migrated / proliferated RPE cells increased and eventually regressed, with a complete resolution in about 40% of the eyes. The intraretinally located RPE cells lost their immunoreactivity for typical retinoid markers and became immunoreactivity positive for immune markers. This aberrant immunoreactivity of the migrated / proliferated RPE cells extended to all abnormal phenotypes, with some of the RPE cells moving into the direction of, and contacting, retinal capillaries. The observation made by Cao et al. agree with findings obtained in other investigations in which activated RPE cells and RPE cells in association with AMD-related fibrosis have been described (Zanzottera et al. 2015; Balaratnasingam et al. 2017; Chen et al. 2022; Curcio et al. 2017). In a similar manner, Jonas and colleagues recently observed, that almost all eyes with the intermediate or late stage of AMD and with macular hyperpigmentations showed intraretinal hyperreflective bodies in optical coherent tomographic images at locations corresponding to the positions of the macular hyperpigmentations. The intraretinal hyperreflective bodies corresponded to intraretinally located RPE cells. Confirming the study conducted by Cao and colleagues, Jonas et al. histologically found intraretinally migrated / proliferated RPE cells in eyes with AMD (and other disorders) (own data) (Fig. 1). Based on the clinical studies, the presence of intraretinally migrated / proliferated RPE cells, corresponding to ophthalmoscopically detectable irregular hyperpigmentations in the macular region in the intermediate stage of AMD, is a prognostically negative sign for further progression of the disease (Mitchell et al. 2018; Fleckenstein et al. 2018; Guymer et al. 2020; 2023; Sadda et al. 2018; Spaide et al. 2018). The intraretinally located RPE cells may be considered as an intraretinal scar-forming process in the intermediate stage of AMD. It is therefore therapeutically useful to prevent the unwanted migration / proliferation of macular RPE cells into the retina.

In the case of the exudative or neovascular type or stage of AMD, the RPE cell proliferations are characterized by the formation of a subretinal scar in the macular region, with the proliferated RPE cells often still having contact with, or producing, a PAS (periodic acid-Schiff-staining)-positive basal membrane. The finding that the proliferating RPE cells still produce their basal membrane within the subretinal scar led to the notion that the RPE cell transformation may best be described as a fibrous pseudo-metaplasia, with the RPE cells still keeping some of their basic epidermal characteristics (i.e., the production of their basal membrane) (Jonas et al. 2021). The exudative or neovascular type of AMD is also characterized by a growth of newly formed blood vessels originating in the choroid and extending through Bruch's membrane into the space beneath the RPE, or after breaking through the RPE, extending into the subretinal compartment in the macular region of the ocular fundus. The clinical introduction of intravitreally applied vascular endothelial growth factor (VEGF) antibodies for the therapy of the neovascular aspect of exudative AMD has been a major step forward in reducing and or even completely stopping the neovascularization. It leads to a slowing of visual decline or even to a temporary increase in visual function (Rosenfeld et al. 2006, Martin et al. 2011).

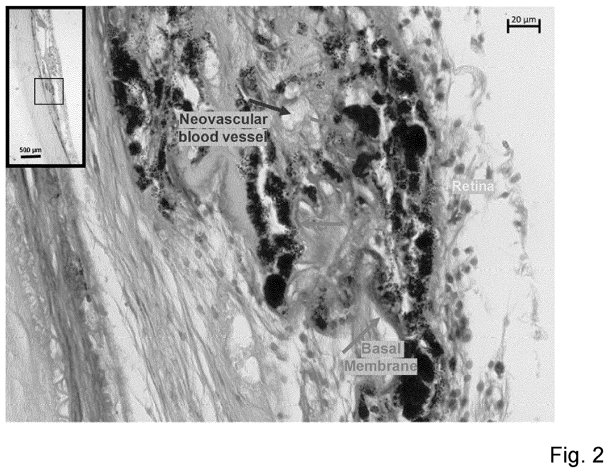

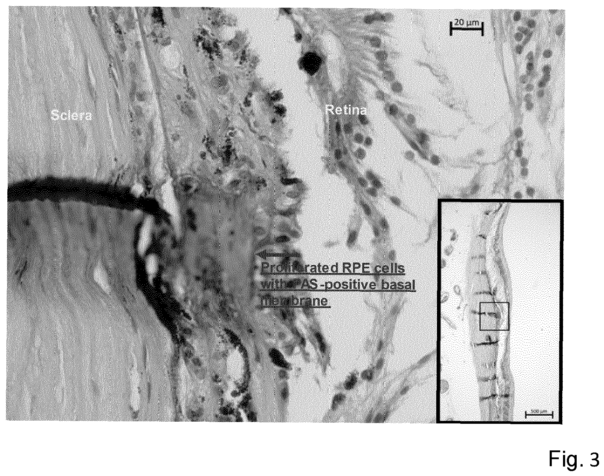

Despite a high number of studies addressing subfoveal neovascularization, only few investigations have been directed so far at the second part of exudative AMD, i.e., the unwanted proliferation, migration and metaplasia of the RPE cells. The proliferation, migration and metaplasia of RPE cells is one of the main features of the exudative form of AMD and leads to the formation of a subretinal scar in the foveal region. The scar separates the photoreceptors from the nourishing choriocapillaris and the scar formation also leads to a loss of normal RPE cells physiologically serving the photoreceptors in their metabolism, in particular in the process of phagocytizing shed photoreceptor outer segments and in recycling photopigments (Rosenfeld et al. 2006, Martin et al. 2011) (Fig. 2,3).

Since the intravitreally applied drugs used so far, such as ranibizumab, bevacizumab, aflibercept, and faricimab, are highly specific for the inhibition of VEGF and some additional other growth factors, such as placental growth factor (in the case of aflibercept) and angiopoietin 2 (in the case of faricimab), none of them has a sizeable direct influence on the proliferation, migration and metaplasia of the RPE. The RPE cells differ from the vascular endothelium and vascular pericytes in many aspects, including its embryological origin (neuro-ectodermal origin versus mesodermal origin) and expression of receptors on their cell surfaces.

### Polypoidal choroidal vasculopathy (PCV)

PCV is another disease located in the macular region and which is characterized by a subretinal choroidal neovascularization accompanied by a subretinal proliferation and scar formation. It shares clinical features with exudative AMD with similarities in the treatment including the intravitreal application of anti-VEGF drugs. In addition, a laser-based photodynamic therapy can be applied. Similar as in AMD, one of the main problems in the therapy of PCV with marked consequences for visual prognosis is the unwanted proliferation of tissue, including RPE cells, beneath the RPE layer or on top of the RPE layer beneath the retina in the macular region. As in AMD, no therapy has been available yet for addressing this unwanted proliferation. The unwanted proliferation leads to a scar increasing the distance between the choroid and the photoreceptors nourished by the choroid and leads to a loss of function of the RPE cells and photoreceptors.

### Myopic macular degeneration with macular neovascularization-associated proliferation of RPE cells and subretinal scar-formation

Also in myopic macular degeneration, a macular neovascularization can occur, originating in the choroid and extending into the compartment beneath the RPE and, after breaking through the RPE layer, reaching the subretinal space (Fig. 2, 3). As in exudative AMD, also in myopic macular neovascularization the RPE cells can proliferate, migrate and undergo a metaplasia or pseudo-metaplasia, resulting in a subretinal scar formation. Upon light-microscopical histology, this subretinal scar consists of proliferated and migrated RPE cells (Jonas et al. 2021).

### Other diseases

Besides exudative AMD, myopic macular neovascularization and PCV there are other diseases, located in the macular region and characterized by a neovascularization, originating in the choroid and extending into the sub-RPE compartment and/or subretinal space. These disorders include traumatic choroidal ruptures, to cite only one example. As in exudative AMD, myopic macular neovascularization and PCV, also these diseases are accompanied by an unwanted proliferation of RPE cells leading to an unwanted scar in the compartment beneath the RPE layer and/or in the subretinal space.

A further disease involving unwanted migration, proliferation, and metaplasia of retinal pigment epithelium (RPE) cells is proliferative vitreoretinopathy (PVR). PVR is a disease that develops as a complication of retinal defects and rhegmatogenous retinal detachment. PVR occurs in about 5-10% of patients undergoing primary retinal detachment surgery and renders surgical repair of rhegmatogenous retinal detachment difficult. PVR is caused by cells such as the RPE cells, invading the vitreous cavity through the retinal defect and forming a scar-like tissue in the vitreous.

This process includes the formation of membranes on the retinal surface with the sequels of a stiffening and contraction of the retina and formation of unattachable retinal folds. PVR can be treated with vitreoretinal surgery with removal of the vitreous body and membranes. By unfolding the retina, the latter can be re-attached. The visual outcome of the surgery can however be limited. A number of studies have explored various possible adjunctive medical agents applied intraocularly for the prevention and treatment of PVR, such as methotrexate or 5-fluorouracile, although none have yet been approved for intravitreal use (Schaub et al. 2022).

With a lack of the possibility of addressing the proliferative part of exudative AMD, of myopic macular neovascularization, of PCV or of any other disease located in the macular region and characterized by a neovascularization, originating in the choroid and extending into the sub-RPE compartment and/or into the subretinal space, with a lack of the possibility of addressing the unwanted intraretinal migration / proliferation of RPE cells in intermediate AMD, and with the lack of an efficient prevention of an intravitreal scar formation in the eyes with a rhegmatogenous retinal detachment, there is a need for a pharmaceutical composition to reduce the unwanted migration, proliferation, and metaplasia or pseudo-metaplasia of RPE cells, in an attempt to prevent the progression of the ocular diseases mentioned and to preserve vision in affected patients.

### SUMMARY OF THE INVENTION

The present invention is *inter alia* based on the finding that pharmaceutical compositions containing EGFR blockers or blockers of agents stimulating the EGF receptor have an impeding effect on the proliferation of retinal pigment epithelium (RPE) cells *in vitro* and *in vivo,* and that the intravitreal application of an EGFR blocker has been well tolerated in patients without observed specific side-effects. It is also based on findings that the repeated intravitreal application of blockers of agents stimulating the EGF receptor has been well tolerated when intraocularly injected in guinea pigs, rabbits and monkeys (Bikbov et al. 2022; Dong et al. 2020; Dong et al. 2022). By targeting EGFR signaling, the pharmaceutical composition can effectively reduce or inhibit the processes in the RPE cells that contribute to the development and progression of intermediate or exudative AMD, myopic macular neovascularization, PCV or any other disease located in the macular region and characterized by a neovascularization, originating in the choroid and extending into the sub-RPE compartment and/or into the subretinal space. In particular, it is understood that the blocking of EGFR signaling has an impact on unwanted migration, proliferation, and metaplasia or pseudo-metaplasia of RPE cells. In the following the term metaplasia refers to both metaplasia and pseudo-metaplasia. Due to the postulated mechanism, it can be assumed that the pharmaceutical composition will have the same effect on other diseases that are associated with unwanted migration, proliferation, and metaplasia of RPE cells, such as proliferative vitreoretinopathy (PVR).

Thus, the invention provides a pharmaceutical composition for use in the treatment of diseases involving unwanted migration, proliferation, and metaplasia of RPE cells in a subject. This composition comprises at least one epidermal growth factor receptor (EGFR) antagonist.

### FIGURES

- **Fig. 1**: shows a clinical photograph and optical coherent tomographic images of an eye with intermediate stage of AMD and macular hyperpigmentation, with the hyperpigmentation on the fundus photograph (yellow arrow) corresponding to a plume-like intraretinal hyperreflective body (or proliferating retinal pigment epithelium cells) in the outer nuclear layer (yellow arrow), apparently emerging out of the retinal pigment epithelium line.
- **Fig. 2**: shows a histo-photograph of a highly myopic eye with pathologic myopia and myopic macular neovascularization, demonstrating the subretinal proliferation of retinal pigment epithelium cells in macular region.
- **Fig. 3**: shows a histo-photograph of a highly myopic eye with pathologic myopia and myopic macular neovascularization, demonstrating the subretinal proliferation of retinal pigment epithelium cells in macular region.

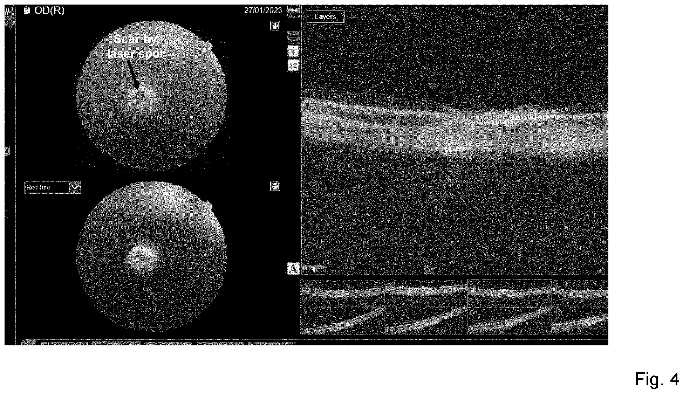

- **Fig. 4**: shows a fundus photograph and optical coherence tomographic image of the fundus of a rabbit eye 15 days after the application of a laser spot twice at the same fundus region; yellow arrow; laser-induced area with depigmentation; red arrow; area with hyperpigmentation within the depigmented area.

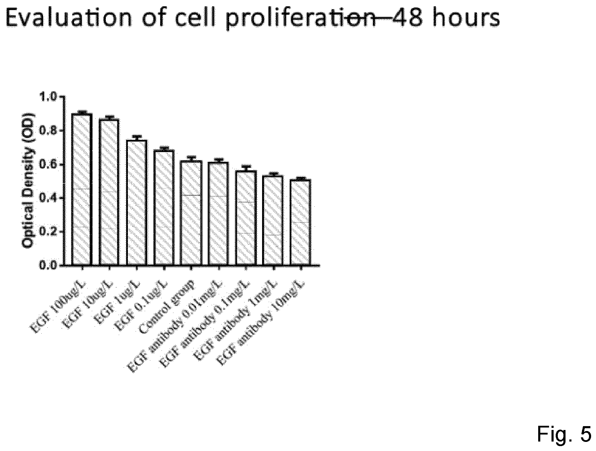

- **Fig. 5**: shows a graph with the results of a retinal pigment epithelium (RPE) cell culture in which epidermal growth factor (EGF) when added to the culture medium increased the proliferation of RPE cells in a dose-dependent manner, while the EGF antibody decreased the proliferation of RPE cells in a dose-dependent manner

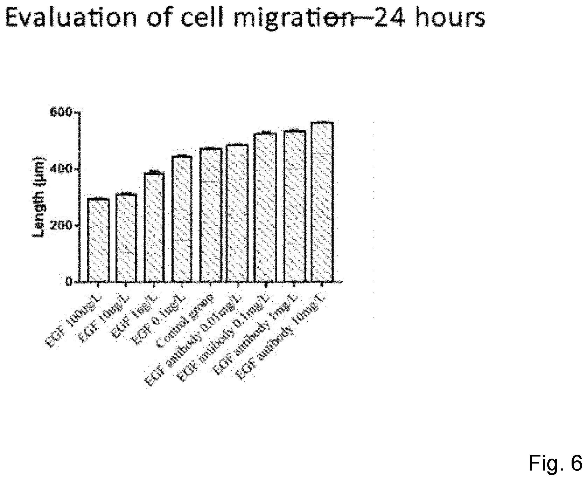

- **Fig. 6**: shows a graph with the results of a retinal pigment epithelium (RPE) cell culture in which the length of a scratch at study end was significantly shorter (i.e., more covered by migrating RPE cells), if epidermal growth factor (EGF) had been added to the culture medium, in contrast to a longer scratch, if EGF antibody had been added. The results suggest a dose-dependent increase in RPE migration by EGF and a dose-dependent decrease in RPE migration by EGF blockers.

### DETAILED DESCRIPTION OF THE INVENTION

The present invention is directed to pharmaceutical compositions for use in the treatment of diseases involving unwanted migration, proliferation, and metaplasia of RPE cells in a subject, comprising at least one EGFR antagonist.

The invention is particularly useful for treating diseases involving unwanted migration, proliferation, and metaplasia of RPE cells in a subject. This pathological process can lead to, and/or can be associated with, the formation of fibrous and fibrovascular membranes, subretinal neovascularization, intraretinal RPE cell migration / proliferation, and retinal detachment, causing severe damage to the retina and compromising vision. Examples of such diseases are intermediate stage AMD, exudative/neovascular/proliferative AMD, macular neovascularization in pathologic myopia, PCV, any other disease located in the macular region and characterized by a neovascularization, originating in the choroid and extending into the sub-RPE compartment and/or into the subretinal space, and PVR.

In the context of the invention, the wet or exudative/neovascular/proliferative AMD is referred to as "exudative AMD".

An "EGFR antagonist" is any protein or other molecule that has the capability of blocking EGFR or an EGFR activator, such as an EGF family member, or eliminating the EGFR expression, so that the activity of the downstream signaling pathways of EGFR is reduced.

The EGFR antagonist may decrease the EGFR signaling by at least 10%, at least 20%, at least 30%, at least 40%, at least 50%, at least 60%, at least 70%, at least 75%, at least 80%, at least 85%, at least 90%, at least 92.5%, at least 95%, at least 97%, at least 98%, at least 98.5%, at least 99%, at least 99.25%, at least 99.5%, or at least 99.75% as compared to normal physiologic levels.

There are a variety of methods known in the art to quantify a decrease in EGFR signaling, such as Western blotting, ELISA, immunohistochemistry and cell-based assays.

Western blotting can be used to detect the expression levels of EGFR and its downstream signaling proteins in cells or tissues. By comparing the levels of these proteins in control and treated samples, the skilled person can assess the decrease in EGFR signaling. ELISA is a quantitative technique that can be used to measure the amount of EGFR ligands or other signaling proteins in a sample. By comparing the levels of these proteins in control and treated samples, the skilled person can determine the extent of the decrease in EGFR signaling. Immunohistochemistry can be used to visualize the location and distribution of EGFR and its downstream signaling proteins in cells or tissues. By comparing the staining intensity or pattern of these proteins in control and treated samples, the skilled person can assess the decrease in EGFR signaling. Cell-based assays can be used to measure the activity of EGFR and its downstream signaling pathways in response to ligand stimulation or other stimuli. By comparing the response of cells in control and treated samples, the skilled person can determine the extent of the decrease in EGFR signaling.

It is noted that the EGFR belongs to the group of ErbB receptors (ErbBs), which consists of four transmembrane receptors belonging to the receptor tyrosine kinase (RTK) superfamily and includes EGFR (ErbB1/HER1), ErbB2/Neu/HER2, ErbB3/HER3, and ErbB4/HER4 (see Bublil et al. 2007). All four ErbBs have a common structure, with a large extracellular ligand-binding region, a single membrane-spanning region, a homologic cytoplasmic protein tyrosine kinase domain and a C-terminal tail with multiple phosphorylation sites.

The extracellular regions of EGFR family members contain two homologous ligand binding domains (domains I and III) and two cystine rich domains (domains II and IV) (Ferguson 2008.

The mRNA sequence encoding is identified by NCBI Reference Sequence: NM_001346897.2 (SEQ ID NO: 1). The amino acid sequence of EGFR is identified by P00533 of UniprotKB, sequence version 2, entry 285 (SEQ ID NO: 2).

In the dimeric complexes formed, for instance, when EGF or TGFα bind to the first three domains of EGFR, the growth factor binding sites are situated far from the dimer interface and do not directly contribute to the contacts within the dimer. All contacts across the dimer interface are mediated by domain II of the receptor - making these dimers 'receptor-mediated' rather than 'ligand-mediated' (Schlessinger 2002). A beta hairpin, referred to as the 'dimerization arm', in domain II makes extensive contacts with the domain II of its binding partner.

The activation of EGFR is a crucial step in many cellular signaling pathways and comprises the steps of a) ligand binding, b) receptor dimerization; c) activation of the intracellular kinase domain; d) recruitment of signaling molecules; and e) signal propagation and cellular response.

Specifically, the activation of EGFR starts with the binding of an EGF family ligand, such as epidermal growth factor (EGF), transforming growth factor-alpha (TGF-alpha), or amphiregulin, to the extracellular domain of the receptor. This interaction is highly specific due to the complementary structure and charge distribution between the ligand and the receptor's binding pocket.

Upon ligand binding, the EGFR undergoes a conformational change that promotes the formation of receptor dimers. These dimers can either be homodimers (two identical EGFR molecules) or heterodimers (EGFR paired with another member of the ErbB receptor family, such as ErbB2, ErbB3, or ErbB4).

The dimerization brings the intracellular kinase domains of the two receptor molecules into close proximity. This spatial arrangement enables the trans-autophosphorylation of specific tyrosine residues in the cytoplasmic tail of each receptor molecule, which activates the receptor's intrinsic kinase activity.

The phosphorylated tyrosine residues in the cytoplasmic tail of the receptor serve as docking sites for various signaling molecules, such as Src homology 2 (SH2) and phosphotyrosine binding (PTB) domain-containing proteins. This recruitment initiates downstream signaling cascades, including the mitogen-activated protein kinase (MAPK) pathway, the phosphoinositide 3-kinase (PI3K)/Akt pathway, and the phospholipase C-gamma (PLCγ) pathway.

The activation of these downstream signaling pathways ultimately leads to changes in gene expression, protein synthesis, and post-translational modifications, which in turn regulate various cellular processes like proliferation, differentiation, migration, and survival.

The EGFR antagonists may include, but are not limited to, an antibody-based molecule capable of binding to EGFR and decreasing EGFR signaling; an antibody-based molecule capable of binding to an EGF family member and decreasing EGFR signaling; a small molecule EGFR tyrosine kinase inhibitor; a peptide inhibitor that is capable of binding to EGFR and decreasing EGFR signaling; an antibody-based molecule capable of binding to an EGF family member and decreasing EGFR signaling; a small interfering RNA (siRNA) agent capable of reducing the expression of EGFR; a CRISPR/Cas9 construct capable of knocking out the EGFR gene, which will directly address the EGFR; and an mRNA molecule encoding an antibody-based molecule capable of binding to EGFR and decreasing EGFR signaling or encoding a peptide inhibitor that is capable of binding to EGFR and decreasing EGFR signaling.

The antibody-based molecule may in particular exert its function of decreasing the EGFR signaling by blocking the binding of the EGF family members to EGFR. The antibody-based molecule may either bind the EGFR directly or a member of the EGF family. Blocking the binding of the EGF family members may, for example, be achieved by binding to the ligand binding domains (I and III) of the extracellular region of EGFR. Alternatively, it may be achieved by binding to the EGFR binding motif of the EGF family members.

The antibody-based molecule may exert its function of decreasing the EGFR signaling by inhibiting the dimerization of EGFR or by inhibiting the tyrosine kinase activity of EGFR. Inhibiting the dimerization of EGFR may, for example, be achieved by binding to the dimerization arm of domain II of the extracellular region. Inhibiting the tyrosine kinase activity may, for example, be achieved by binding to the intracellular tyrosine kinase domain.

According to one embodiment, the antibody-based molecule is selected from an antibody-based molecule blocking the binding of the EGF family members to EGFR, an antibody-based molecule inhibiting the dimerization of EGFR, or an antibody-based molecule inhibiting the tyrosine kinase activity of EGFR.

Besides EGF itself other family members comprise: Heparin-binding EGF-like growth factor (HB-EGF), transforming growth factor-α (TGF-α), amphiregulin (AR), epiregulin (EPR), epigen, betacellulin (BTC), neuregulin-1 (NRG1), neuregulin-2 (NRG2), neuregulin-3 (NRG3), and neuregulin-4 (NRG4). Members of the EGF family have highly similar structural and functional characteristics. The ability of the EGF-family members to bind EGFR is based on the common presence of one or more repeats of the "EGFR binding motif" with the conserved amino acid sequence:
**C**X₇**C**X_{**4**-5}**C**X₁₀₋₁₃**C**X**C**X₅**G**X**RC** (SEQ ID NO: 3)
with X representing any amino acid.

This sequence contains six cysteine residues that form three intramolecular disulfide bonds. Disulfide bond formation generates three structural loops that are essential for high-affinity binding between members of the EGF-family and their cell-surface receptors.

According to one embodiment the EGFR antagonist is an antibody-based molecule capable of binding to an EGF family member selected from EGF, HB-EGF, TGF-α, AR, EPR, epigen, BTC, NRG1, NRG2, NRG3, NRG4 and decreasing EGFR signaling.

According to one embodiment, the antibody-based molecule is selected from an antibody, an antibody fragment, an antibody mimetic.

According to one embodiment, the antibody is preferably selected from panitumumab, cetuximab, nimotuzumab, matuzumab, pertuzumab and trastuzumab. Cetuximab (Erbitux^{®}) is a chimeric (mouse/human) monoclonal antibody that targets the extracellular domain of EGFR. By binding to the receptor, it prevents the binding of endogenous ligands and subsequent receptor activation, thus inhibiting downstream signaling pathways. Cetuximab is used for the treatment of metastatic colorectal cancer (mCRC) with wild-type KRAS and in combination with radiation therapy or chemotherapy for the treatment of locally or regionally advanced head and neck squamous cell carcinoma (HNSCC). It is also used for recurrent or metastatic HNSCC that has progressed following platinum-based therapy (see e.g., Cunningham et al. 2004). Panitumumab (Vectibix^{®}) is a fully human monoclonal antibody that also targets the extracellular domain of EGFR, blocking ligand binding and receptor activation. It is used as a single agent for the treatment of metastatic colorectal cancer (mCRC) with wild-type KRAS when disease progression has occurred following chemotherapy. Panitumumab has also been investigated in combination with other therapies for the treatment of various solid tumors (see e.g., Hecht et al. 2007). Nimotuzumab (Theraloc^{®}, BIOMAb EGFR^{®}) is a humanized monoclonal antibody that targets the extracellular domain of EGFR, similar to cetuximab and panitumumab. It has been approved for use in some countries for the treatment of head and neck cancer, glioma, and other cancers (see e.g., Ramakrishnan et al. 2009). Matuzumab (EMD 72000^{®}) is another humanized monoclonal antibody targeting EGFR. It has been tested in clinical trials for the treatment of various solid tumors, including non-small cell lung cancer (NSCLC), colorectal cancer, and ovarian cancer. However, it did not show significant clinical benefit in these trials, and its development has been discontinued (see e.g., Vanhoefer et al. 2004). Pertuzumab (Perjeta^{®}) is a humanized monoclonal antibody that targets the extracellular dimerization domain of the human epidermal growth factor receptor 2 (HER2), another member of the ErbB family. It is used in combination with Trastuzumab (Herceptin^{®}) and chemotherapy for the treatment of HER2-positive breast cancer (see e.g., Swain et al. 2015). Trastuzumab is a humanized monoclonal antibody that targets HER2. It is widely used for the treatment of HER2-positive breast cancer and HER2-positive metastatic gastric or gastroesophageal junction adenocarcinoma (see Piccart-Gebhart et al. 2005).

According to one embodiment, the EGFR antagonist is an antibody fragment. According to one embodiment, the antibody fragment is selected from the group consisting of Fab fragments, F(ab')₂ fragments and Fab' fragments. Fab fragments are antibody fragments that consist of the variable regions of the heavy and light chains of an antibody, as well as the first constant region of the heavy chain. Fab fragments can be produced by enzymatic digestion of full-length antibodies with papain. F(ab')₂ fragments are antibody fragments that consist of two Fab fragments linked together by a disulfide bond. F(ab')₂ fragments can be produced by enzymatic digestion of full-length antibodies with pepsin. Fab' fragments are antibody fragments that consist of the variable regions of the heavy and light chains of an antibody, as well as a portion of the constant region of the heavy chain. Fab' fragments can be produced by enzymatic digestion of full-length antibodies with papain followed by reduction of the disulfide bonds connecting the heavy chains. These fragments are commonly used in research and diagnostic applications and methods for the generation of antibody fragments are not particularly limited and are known in the art.

According to one embodiment, the EGFR antagonist is an antibody mimetic. The antibody mimetic according to the invention may be selected from the group consisting of single-chain variable fragments (scFv), single-domain antibodies, affibodies, affilins, affimers, affitins, anticalins, DARPins, monobodies, and peptide aptamers.

Single-chain variable fragments (scFv) are a type of antibody fragment that consist of the variable domains of the heavy and light chains of an antibody linked together by a short peptide linker. They can be produced in bacteria, yeast, or mammalian cells. Single-domain antibodies, also known as nanobodies, are antibody fragments that consist of a single variable domain from either the heavy or light chain of an antibody. They are smaller and more stable than traditional antibodies. Affibodies are small protein scaffolds that are engineered to bind to specific targets with high affinity and specificity. They are based on the B-domain of protein A, which is a natural ligand for the Fc region of antibodies. Affilins are a type of small protein scaffold that are engineered to bind to specific targets with high affinity and specificity. They are based on the cystatin protein family, which are natural protease inhibitors. Affimers are a type of protein scaffold that are engineered to bind to specific targets with high affinity and specificity. They are based on a protein called staphylococcal nuclease. Affitins are a type of protein scaffold that are engineered to bind to specific targets with high affinity and specificity. They are based on a protein called ExbB, which is involved in iron transport in bacteria, and can be used in research, diagnostic, and therapeutic applications. Anticalins are a type of protein scaffold that are engineered to bind to specific targets with high affinity and specificity. They are based on a protein called lipocalin, which is involved in the transport of small hydrophobic molecules. DARPins, or designed ankyrin repeat proteins, are a type of protein scaffold that are engineered to bind to specific targets with high affinity and specificity. They are based on the ankyrin repeat protein family, and can be used in research, diagnostic, and therapeutic applications. Monobodies are a type of antibody mimetic that consist of a single protein domain engineered to bind to specific targets with high affinity and specificity. They are based on the fibronectin type III domain, and can be used in research, diagnostic, and therapeutic applications. Peptide aptamers are a type of protein scaffold that consist of a short peptide sequence that is engineered to bind to specific targets with high affinity and specificity. They are often generated using phage display or other selection methods, and can be used in research, diagnostic, and therapeutic applications. Respective antibody mimetics and methods for producing the same are not particularly limited and are known in the art.

According to one embodiment, the EGFR antagonist is a small molecule EGFR tyrosine kinase inhibitor TKI. Small molecule TKIs are a class of targeted cancer therapies specifically designed to interfere with the enzymatic activity of the EGFR.

Small molecule TKIs are small molecules that selectively bind to the intracellular kinase domain of EGFR, usually at the ATP-binding site, and block its tyrosine kinase activity. By inhibiting EGFR's kinase activity, these TKIs can disrupt the downstream signaling pathways responsible for tumor growth and survival. As the small molecule TKIs successfully decrease EGFR signaling, they are also suitable for the treatment of diseases involving unwanted migration, proliferation and metaplasia of RPE.

Several small molecule TKIs have been developed to target dysregulated EGFR in various cancers. Examples include gefitinib, erlotinib, lapatinib, icotinib, afatinib, neratinib, dacomitinib, almonertinib, olmutinib, osimertinib, brigatinib, vandetanib, and pyrotinib. Lapatinib (Tykerb^{®} or Tyverb^{®}) is a dual EGFR and HER2 TKI used for the treatment of HER2-positive breast cancer, often in combination with other treatments like capecitabine. Icotinib (Conmana^{®}) is a small molecule EGFR TKI used primarily for the treatment of non-small cell lung cancer (NSCLC) with activating EGFR mutations. Neratinib (Nerlynx^{®}) is a pan-HER inhibitor that targets EGFR, HER2, and HER4, used for the extended adjuvant treatment of early-stage, HER2-positive breast cancer. Dacomitinib (Vizimpro^{®}) is an irreversible, pan-HER inhibitor that targets EGFR, HER2, and HER4, used as a first-line treatment for metastatic non-small cell lung cancer (NSCLC) with EGFR exon 19 deletions or exon 21 L858R substitution mutations. Almonertinib (Ametyn^{®}) is a third-generation small molecule EGFR TKI used for the treatment of advanced non-small cell lung cancer (NSCLC) with EGFR T790M mutations that have progressed on or after EGFR TKI therapy. Olmutinib (BI 1482694 or HM61713) is a third-generation small molecule EGFR TKI that was being developed for the treatment of non-small cell lung cancer (NSCLC) with EGFR T790M mutations. Brigatinib (Alunbrig^{®}) is a TKI that targets ALK (anaplastic lymphoma kinase) and EGFR, used for the treatment of metastatic non-small cell lung cancer (NSCLC) with ALK rearrangements in patients who have progressed on or are intolerant to crizotinib. Vandetanib (Caprelsa^{®}) is a multi-targeted TKI that inhibits EGFR, VEGFR, and RET kinases, used for the treatment of symptomatic or progressive medullary thyroid cancer in patients with unresectable, locally advanced, or metastatic disease. Pyrotinib (Lumitin^{®}) is an irreversible, pan-HER inhibitor that targets EGFR, HER2, and HER4, used for the treatment of HER2-positive breast cancer, often in combination with other treatments like capecitabine. Methods for producing the small molecule EGFR TKIs are not particularly limited and are known in the art.

According to one embodiment, the EGFR antagonist is a peptide inhibitor. According to one embodiment, the peptide inhibitor is selected from a peptide inhibiting the binding of the EGF family members to EGFR, a peptide inhibiting the dimerization of EGFR, or a peptide inhibiting the tyrosine kinase activity of EGFR. The peptide inhibiting the binding of the EGF family members to EGFR may for example bind to the ligand binding domains (I and III) of the extracellular region of EGFR. Alternatively, the peptide inhibiting the binding of the EGF family members to EGFR may bind to the EGFR binding motif of the EGF family members. According to one embodiment the peptide inhibitor has a length of at most 80 amino acids. The peptide inhibitor may have a length of 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, or 80 amino acids. According to one embodiment the peptide inhibitor has a length of at most 60 amino acids. According to one embodiment the peptide inhibitor has a length of at most 40 amino acids. According to one embodiment the peptide inhibitor has a length of at most 20 amino acids. Peptide inhibitors are for example described in Ahsan et al. 2014. Methods for producing the peptide inhibitors are not particularly limited and are known in the art.

According to one embodiment, the EGFR antagonist is a small interfering RNA (siRNA) agent capable of reducing the expression of EGFR. siRNA is a type of double-stranded RNA molecule, typically around 21-23 nucleotides in length, that is involved in RNA interference (RNAi) pathway. RNAi is a natural mechanism that cells use to silence gene expression by degrading messenger RNA (mRNA) molecules that carry genetic information from DNA to the ribosomes where proteins are synthesized. siRNA molecules are designed to be complementary to specific mRNA sequences, leading to their degradation and subsequent inhibition of protein synthesis. In siRNA, the antisense strand is the strand of RNA that is complementary to the target mRNA molecule, and it is the one that is responsible for base-pairing with the mRNA to induce its degradation. The antisense strand of siRNA is typically 21-23 nucleotides in length, and it is usually designed to be fully complementary to the target mRNA sequence, with the exception of a two-nucleotide overhang at the 3' end of the antisense strand. This overhang is important for determining the specificity of the siRNA and for preventing off-target effects.

According to one embodiment, the siRNA agent contains an antisense strand, and this antisense strand is 21, 22 or 23 nucleotides in length. Moreover the antisense strand is designed to be fully complementary to a stretch of the EGFR mRNA sequence (SEQ ID NO: 1). The antisense strand further comprises an overhang of at least two nucleotides at the 3' end that is not compatible with the stretch of SEQ ID NO: 1.

According to one embodiment, the EGFR antagonist is an mRNA molecule. According to one embodiment, the mRNA molecule encodes an antibody. According to one embodiment, the antibody encoded by the mRNA is selected from panitumumab, cetuximab, nimotuzumab, matuzumab, pertuzumab and trastuzumab. According to one embodiment, the mRNA molecule encodes a peptide inhibitor as described above. Administering an antibody against the EGFR using an mRNA-based approach would involve a slightly different process than traditional mRNA vaccines established by Moderna and BionTech. Instead of encoding an antigen to stimulate an immune response, the mRNA would encode the antibody itself.

In the following, the process is briefly outlined. A) Design and production: The mRNA sequence encoding the desired antibody specifically targeting EGFR as described above is synthesized. This sequence would typically consist of both the heavy and light chains of the antibody, which are optimized for stability, efficient translation, and reduced immunogenicity. B) Delivery system: Similar to the mRNA vaccines, the mRNA encoding the antibody may be encapsulated in lipid nanoparticles (LNPs) to protect it from degradation and facilitate its entry into host cells. C) Administration: The mRNA-antibody construct is injected into the recipient, usually via intramuscular or subcutaneous routes, in the present case using one of the administration routes listed below, e.g., by intraocular application. The mRNA is taken up by cells at the injection site. D) Translation and secretion: Once inside the host cells, the mRNA is released from the LNPs and translated by cellular machinery to produce the antibody. The antibody's heavy and light chains assemble into a functional antibody, which is then secreted from the cell into the extracellular space and subsequently into the intraocular compartment and the bloodstream. E) Binding and neutralization: The synthesized antibody specifically binds to EGFR which may lead to decreasing the receptor's function.

According to one embodiment, the use is a therapeutic or prophylactic treatment. The pharmaceutical composition comprising the EGFR agonist may be administered intravitreally, epicorneally, transcorneally, transsclerally, transconjunctivally, subconjunctivally, intraocularly or into the Tenon's space.

A further option of the administration is the administration method of PLGA microparticle Densomeres for extended release of biotherapeutic antibodies as described in Peterson et al. 2023. To formulate the Densomeres, an antibody solution is combined with a PLGA solution and processed using a proprietary technique. The Densomere microparticle carriers (DMCs) can be suspended in a range of sizes for injection or inhalation, or shaped into implantable solid forms for local delivery in various tissues. In this study conducted by Peterson and colleagues, DMC suspensions were administered as a single subconjunctival injection in the rabbit corneal model for suppression of neovascular encroachment.

Needles for administering the pharmaceutical composition are known in the art. Possible needle sizes according to the Birmingham wire gauge system are gauge 25, 26, 27, 28, 29, 30, 31, 32, 33, 34. According to one embodiment, the needle size is selected from gauge 28, 29, 30, 31, 32. According to one embodiment, the needle size is gauge 30. Syringes for administering the pharmaceutical composition are known in the art. Suitable syringe sizes are 0.5 ml, 1.0 ml, 2.5 ml or 5 ml. In particular, tuberculin syringes (volume 1 ml), are well suited.

Preferably, the pharmaceutical composition is administered intravitreally. For the intravitreal application, it is suggested to apply anesthesia. In particular, anesthesia of the cornea and conjunctiva is obtained by applying topical anesthetic eye drops (such as oxybuprocain). The external ocular surface as well as the lid margins and the lids are desinfected and a lid speculum is inserted.

The pharmaceutical composition may be injected at different locations of the eye. According to one embodiment, the pharmaceutical composition is injected in the temporal inferior quadrant. According to one embodiment, the pharmaceutical composition is injected posterior to the corneal limbus. According to one embodiment, the pharmaceutical composition is injected through the conjunctiva, sclera and pars plana of the ciliary body into the vitreous cavity.

According to one embodiment, the pharmaceutical composition is injected in a distance of 1 mm to 6 mm posterior to the corneal limbus. The distance may, for example be 1.0 mm, 1.2 mm, 1.4 mm, 1.6 mm, 1.8 mm, 2.0 mm, 2.2 mm, 2.4 mm, 2.6 mm, 2.8 mm, 3.0 mm, 3.2 mm, 3.4 mm, 3.6 mm, 3.8 mm, 4.0 mm, 4.2 mm, 4.4 mm, 4.6 mm, 4.8 mm, 5.0 mm, 5.2 mm, 5.4 mm, 5.6 mm, 5.8 mm, or 6.0 mm. According to one embodiment, the pharmaceutical composition is injected in a distance of 2 mm to 5 mm posterior to the corneal limbus. According to one embodiment, the pharmaceutical composition is injected in a distance of 3 mm to 4 mm posterior to the corneal limbus.

After the needle is withdrawn an ointment containing an antibiotic and an antiinflammatory agent may be applied.

For the case the EGFR antagonist is an antibody-based molecule, the total dosage of the EGFR antagonist may be in the range of 0.1 mg to 6 mg per eye. The total dosage may be 0.1 mg, 0.2 mg, 0.3 mg, 0.4 mg, 0.5 mg, 0.6 mg, 0.7 mg, 0.8 mg, 0.9 mg, 1.0 mg, 1.2 mg, 1.4 mg, 1.6 mg, 1.8 mg, 2.0 mg, 2.2 mg, 2.4 mg, 2.6 mg, 2.8 mg, 3.0 mg, 3.2 mg, 3.4 mg, 3.6 mg, 3.8 mg, 4.0 mg, 4.2 mg, 4.4 mg, 4.6 mg, 4.8 mg, 5.0 mg, 5.2 mg, 5.4 mg, 5.6 mg, 5.8 mg, or 6.0 mg. In a dosage of less than 0.1 mg, no significant therapeutic or prophylactic effect is measured. A dosage above 6.0 mg does not further improve the pharmaceutical effect and increases the risk of side effects. According to one embodiment, the antibody-based molecule is administered in the pharmaceutical composition in a dosage in the range of 0.30 µg to 3 mg. According to one embodiment, the antibody-based molecule is administered in the pharmaceutical composition in a dosage in the in the range of 0.5 mg to 2 mg.

The pharmaceutical composition may be administered at least 2 times, at least 3 times, at least 4 times, at least 5 times, at least 6 times, at least 7 times, at least 8 times, at least 9 times, at least 10 times, at least 15 times, at least 20 times, at least 25 times, at least 30 times, at least 35 times, or at least 40 times.

The administration of the EGFR antagonist is expected to have low side effects. As shown in Examples 3 and 4, neither in the rabbits nor in the guinea pigs, any injection-related effects such as a loss of retinal cells, a change in the intravitally measured optical coherence tomography (OCT) based retinal thickness measurements, an increase in the number of apoptotic retinal cells, a shrinkage or swelling of the ciliary body, and induction of astrogliosis, or changes in the intraocular pressure were noted. In the same manner, there were no signs of intraocular inflammation detected, neither during the intravital examination nor upon histological examinations of the globes. The findings support the notion, that repeatedly intravitreally applied EGFR antagonist does not result in an intraocular inflammatory or toxic effect.

The pharmaceutical composition may be in any suitable dosage form for administration to the patient, for example in form of crystals, a solution or a lyophilisate. According to one embodiment, the pharmaceutical composition is a solution or a lyophilisate.

In a preferred embodiment, the EGFR antagonist or a pharmaceutically acceptable salt thereof is formulated in the pharmaceutical composition with one or more pharmaceutically acceptable excipient(s) and/or carrier(s).

The lyophilized protein may be reconstituted in sterile buffer. Suitable buffers components are, for example citrate or sodium phosphate. Sodium phosphate buffer consists of sodium dihydrogen phosphate (NaH₂PO₄) and sodium dihydrogen phosphate (Na₂HPO₄).

In order to increase the stability of the EGFR antagonist in solution allowing a longer storage time, the buffered solution may be supplemented with stabilizers. Suitable stabilizers include sucrose, dextran, and carrier proteins such as heat inactivated fetal calf serum (FCS) or tissue culture grade bovine serum albumin (BSA). According to one embodiment the lyophilized EGFR antagonist is reconstituted in water for injection. Accordingly, in the pharmaceutical composition, the EGFR antagonist may be solubilized in water for injection.

According to an alternative embodiment, the EGFR antagonist is formulated with sucrose, dextran and a sodium phosphate buffer.

The volume to be injected may range from 30 µL up to 300 µL. For example, the volume may be 30 µL, 40 µL, 50 µL, 60 µL, 70 µL, 80 µL, 90 µL, 100 µL, 120 µL, 140 µL, 160 µL, 180 µL, or 200 µL. According to one embodiment, the volume is in the range of 50 µL up to 100 µL. For volumes of more 100 µL, a paracentesis would be performed to release fluid from the anterior chamber and to reduce the ocular volume before the injection. Anterior chamber paracentesis is a known method for the skilled person.

The treatment may be a one-time treatment. Preferably, the pharmaceutical composition is administered multiple times. The time interval between the administrations may be in the range of 2 days to 12 months. For example, the time interval between the administrations is 2 days, 3 days, 4 days, 5 days, 6 days, 7 days, 2 weeks, 3 weeks, 4 weeks, 5 weeks, 6 weeks, 7 weeks, 8 weeks, 9 weeks, 10 weeks, 11 weeks, 12 weeks, 3 months, 4 months, 5 months, 6 months, 9 months, 12 months. According to one embodiment, the time interval between the administrations is in the range of 2 weeks to 7 months. According to one embodiment, the time interval between the administrations is in the range of 4 weeks to 6 months.

### EXAMPLES

### Example 1 - Treatment of AMD model rabbits

### Methods

The experimental study included rabbits (gray Soviet chinchilla; age: 3-4 months; weight: 2.5-3 kg). All animals were treated in accordance with the ARVO (Association for Research in Vision and Ophthalmology) Statement for the Use of Animals in Ophthalmic and Vision Research. The Ufa Eye Research Institute Biomedical Ethics Committee approved the investigation which is reported in accordance with the ARRIVE (Animal Research: Reporting of In Vivo Experiments) guidelines. The animals purchased from a commercial vendor (Federal State Unitary Enterprise "Scientific and Production Association for Immunological Preparations "Microgen" of the Ministry of Health of the Russian Federation, Ufa, Bashkortostan, Russia) were kept at a constant temperature (22 ± 1 °C) and in a light-controlled environment (lights on from 7 am to 7 pm) with ad libitum access to food and water.

The right eyes of the rabbits underwent a laser-induced coagulation of their posterior pole by applying an argon laser coagulation spot à 500 milli Watt with a spot diameter of 100 µm and a duration of 0.5 seconds twice within an interval of 2 minutes at the same location of the posterior retina. At once after the injection, the rabbits of the study group received an intravitreal injection of 1 mg of panitumumab in 0.10 mL. The left eyes received an intravitreal injection of 0.10 mL of Ringer's solution (Gematek OOO Company, Moscow, Russia). Directly after each injection, we measured the intraocular (IOP) of both eyes. The injections and the retinal laser coagulation, carried out at the same setting, were performed in general anesthesia which was achieved by an intramuscular injection (biceps femoris) of Zoletil (15 mg/kg) (tiletamine mixed with zolazepam; Valdepharm Co., Val-de-Reuil, France) and xylazine (20 mg/kg) (Xyla; Interchemie Werken, De Adelaar B.V., A Waalre, The Netherlands). We additionally applied anesthetic eye drops (0.4% oxybuprocain, Inocain^{®}; Promed Exports, New Delhi, India) topically. The injections were carried out in the temporal upper quadrant at a distance of 3 to 4 mm from the limbus. The technique has been described recently in detail (Bikbov et al. 2020, Bikbov et al. 2022).

The injections of panitumumab and of PBS were repeated twice in intervals of three days. At baseline, at the time points of the re-injections, and at study end three days after the last (third) injection, the animals were re-examined by inspection of the external eye, tonometry (Auto-2Ref/Keratometer HRK-7000A Huvitz Co, Ltd., Gyeonggi-do, Korea), fundus photography (VISUCAM 500, Carl Zeiss Meditec AG, Jena Germany), and optical coherence tomography (OCT) of the laser spot. Using morphometry of the fundus photographs and on the OCT-images, we measured the size of the depigmented area and the size of region with hyperpigmentation within the depigmentated area. The latest examination was performed at study end at 15 days after the laser application (Fig. 4).

Applying a statistical software package (SPSS for Windows, version 27.0, IBM-SPSS, Chicago, IL, USA), we determined the mean values ± their standard deviations of the main outcome parameters, i.e., the size of the depigmentated area in the region of the laser spot application, and the size of the area with hyperpigmentation within the laser spot region. We applied the Wilcoxon-Mann-Whitney test for unpaired samples to compare the parameters between the study and control group. A two-sided P-values was considered statistically significant if it was smaller than 0.05.

### Results

The study included altogether 7 rabbits, randomly assigned to a study group of 3 animals and a control group of 4 animals. Both groups did not differ significantly in body weight *P*<0.05). The area of the laser-induced spot of depigmentation did not vary significantly between both groups (1.74 ± 1.65 mm² versus 1.56 ± 0.42 mm²; P=0.29). The area of hyperpigmentation as a surrogate of RPE proliferation was significantly smaller in the study group than in the control group 0.35 ± 0.19 mm² versus 0.42 ± 0.49 mm²; *P*=0.03). The ratio of hyperpigmented area to the depigmentated area was significantly smaller in the study group than in the control group (0.05 ± 0.04 versus 0.23 ± 0.09; P=0.03). Both groups did not differ significantly in anterior chamber flare or cells as examined by slit lamp-based biomorphometry, vitreous haze, vitreous hemorrhage, ciliary injection, and intraocular pressure.

### Example 2 - Safety of intravitreally administered EGFR antibodies in highly myopic patients with myopic macular degeneration

### Methods

This study was designed as a monocenter phase-1 examination in an open-label, multiple-dose study on safety and tolerability of intravitreally applied Panitumumab in adult highly myopic patients with myopic macular degeneration. The Ethics Committee of the Academic Council of the Ufa Eye Research Institute approved the study (date: 02.11.2021) and confirmed that the study adhered to the tenets of the Declaration of Helsinki (registered trial number: DRKS00027302). Informed written consent was obtained from all study participants. Recruitment began in November 2021.

Inclusion criteria were an age of 50+ years, an axial length of ≥26.5mm, myopic macular degeneration stage 4 with foveal patchy atrophies as defined by the META-analysis for Pathologic Myopia Study Group, best corrected visual acuity (BCVA) ≥1.0 logMAR (logarithm of the minimal angle of resolution) (20/200 Snellen equivalent), and clear optic media to allow imaging of the macula and optic nerve head (Ohno-Matsui et al. 2015).

Exclusion criteria were previous vitreoretinal or retinal surgeries (except for peripheral retinal laser coagulation), previous intravitreal medical therapy with vascular endothelial growth factor (VEGF) inhibitors during the last three months before inclusion into the study, active choroidal neovascularization, subretinal or intraretinal edema in the macular region, pregnancy, lack of reliable contraception unless menopause had occurred, known intolerance or hypersensitivity against panitumumab (Vectibix^{®}) or its ingredients, participation in another clinical trial (parallel or within a cooling off period of a previous trial), and missing ability to understand and sign a written informed consent.

The study population consisted of three dose cohorts with increasing doses of Panitumumab (0.6 mg, 1.2 mg or 1.8 mg). Each dose-step was separated by a comprehensive review of safety and tolerability of the injections considering adverse events and the results of detailed ophthalmological examinations including the sclera and cornea, the conjunctiva with the injection site, the lens and the ocular fundus in the period after the injections. The inclusion of 11 patients into the study was assessed to be sufficient since no formal statistical comparisons were planned, and the minimum of 3 participants per cohort was regarded sufficient to proceed to a higher dose level as long as no dose-limiting findings were noted.

At baseline, the patients underwent a detailed ophthalmological examination including automated and subjective refractometry (performed three times at different time points, which could be during the same day), measurement of BCVA (using the standard ETDRS (Early Treatment of Diabetic Retinopathy Study) testing protocol), perimetry (PTS-1000, Optopol Technology, Zawiercie, Poland), laser interferometric biometry for measurement of axial length (AL-Scan; Nidek Co., Ltd., Gamagori, Japan), slit lamp-based examination of the anterior and posterior segment with special attention on intraocular inflammatory signs, tonometry (Tonoref III, Nidek Co., Ltd., Japan), photography of the fundus, optical coherence tomography (OCT) of the macula and optic nerve head, assessment of the fundus autofluorescence (SS-OCT (DRI-OCT, Triton; Topcon Inc., Tokyo, Japan)), and electroretinography ("Neiro-ERG" Neirosoft, Russia). This whole series of examinations were repeated at the time of every re-injection and at the end of the observation period. This entire series of examinations were repeated at days 1 and 7, and at 1 and 2 months after the intravitreal injection, and at the time of every re-injection and at the end of the observation period.

The intravitreal Panitumumab injections were performed in the operation theater under sterile conditions in topical anesthesia, similar to conventional intravitreal injections of anti-VEGF (vascular endothelial growth factor) drugs. In a first step, a paracentesis was performed to release about 0.1 to 0.2 mL of aqueous humor to reduce the intraocular volume and to create space for the following intravitreal injection of panitumumab (Vectibix^{®}). The sampled aqueous humor samples were collected and deeply frozen for later biochemical analyses. The intravitreal injection was performed transconjunctivally in the temporal inferior quadrant in a distance of 3.0 to 3.5 mm from the limbus. Care was taken that before the injection, the conjunctiva was slightly shifted so that the conjunctival perforation site was not identical with the scleral perforation site. The injections in volumes of 60 µL (0.6 mg Panitumumab), 120 µL (1.2 mg Panitumumab) and 180 µL (1.8 mg Panitumumab) were directed into the center of the vitreous cavity. Depending on the intraocular pressure at the end of the injection and of the alignment of the scleral perforation site, some fluid from the vitreous cavity spilled back under the conjunctiva. Testing of the visibility of hand movements followed the intravitreal injections. An ointment containing a topical antibiotic and a topical steroid (levofloxacini 0.5%, Lekko, Russia dexamethasone 0.1%. «Belmedpreparaty», Republic of Belarus) was applied.

### Dose considerations:

In animal studies, a dose of 20 µg EGFR antibody (molecular weight: 175 kDa; #2232, Cell Signaling Technology, Danvers, MA, USA) was used in guinea pig eyes with a diameter of about 8 mm, corresponding to an ocular volume of about 268 mm³ (Jiang et al. 2017; Dong et al. 2019; Dong et al. 2020; Dong et al. 2022). It related to an intraocular concentration 0.07 µg EGFR antibody / mm³). It corresponded to a dose of 0.72 mg EGFR antibody in a highly myopic adult human eyes with an axial length of 27 mm or an intraocular volume of approximately 10.306 mm³, assuming a spherical eye shape. Taking into account the molecular weight of Panitumumab (144.3 kDa) compared to the molecular weight of the antibody used in the guinea pig study (175 kDa), the equimolar dose of Panitumumab is 0.59 mg. This dose of 0.6 mg Panitumumab is approximately 1/700 of the systemically applied dose of Panitumumab (Vectibix^{®}) administered intravenously every two weeks for oncological indications according to the SmPC (summary of product characteristics) (6 mg Panitumumab / kg body weight or about 420 mg Panitumumab for a patient with a body weight of 70 kg) (Douillard et al. 2013). Panitumumab (Vectibix^{®}) is delivered by the pharmaceutical company in a concentration of 100 mg/5 mL (or 20 mg/mL) and has to be diluted to a concentration of 10 mg/mL. The doses of 0.6 mg, 1.2 mg and 1.8 mg Panitumumab for intravitreal application thus led to an injection volume of 60 µL, 120 µL and 180 µL, respectively.

Based on the experiences gathered worldwide with the intravitreal application of bevacizumab (Avastin^{®}) used for the treatment of neovascular macular degeneration and other retinal diseases, an intravitreally injected dose of 1.25 mg bevacizumab (molecular weight: 149 kDa), corresponding to a molecular dose of 8 nmol bevacizumab, is intraocularly and systemically well tolerated (Martin et al. 2011). In a similar manner, the intravitreal application of ranibizumab (Lucentis^{®}) (also for the therapy of exudative macular degeneration) in a dose of 0.50 mg ranibizumab (molecular weight: 48 kDa) (corresponding to a molecular dose of 10 nmol ranibizumab) is intraocularly and systemically well tolerated (Martin et al. 2011). A dose of 0.6 mg panitumumab (molecular weight: 144.3 kDa) corresponds to 4 nmol panitumumab, which is about half of the molar dose of ranibizumab injected intravitreally. In oncology, Avastin^{®} (bevacizumab) is given intravenously every three weeks at a dose of 15 mg / kg body weight (or 1.2 g bevacizumab at a body weight of 80 kg) Hurwitz et al. 2004). The routinely and intravitreally applied dose of bevacizumab of 1.25 mg is thus about 1/1,000 of the intravenously applied dose of bevacizumab in oncology.

### Results

A total of 11 patients (2 men, 9 women) with a mean age of 66.8 ± 6.3 years (median: 65.7 years; range: 57.6 to 80.2 years) were included. Mean best corrected visual acuity was 1.62 ± 0.47 logMAR (median: 1.52 logMAR; range: 1.00 to 2.30).

All participants treated in the study self-identified as Russian (n=6), Tartar (n=3) or of other ethnicity (n=2). The mean body height was 1.67 ± 0.10 m (median: 1.70 m; range: 1.46 - 1.80 m), mean body weight was 66.6 ± 8.2 kg (median: 65.0 kg; range: 53-80 kg), and mean body mass index was 24.0 ± 2.4 kg/m² (median: 24.7 kg/m²; range: 20.1 -27.6 kg/m²). The follow-up ranged between one week and 11.9 months (mean: 5.6 ± 3.4 months; median: 6.6 months).

All patients received at least one intravitreal injection of panitumumab. The patients received the panitumumab injections in doses of 0.6 mg (4 eyes; 1x1 injection, 3x2 Injections), 1.2mg (4 eyes; 1x1 injection, 2 x 2 injections; 1x3 injections) and 1.8 mg (3 eyes; 1x1 injection, 2x2 injections), respectively (Table 1). The mean interval between the first injection and second injection was 3.6 ± 1.8 months (medium: 2.6 months; range: 2.1 to 6.3 months), and the interval between the second injection and third injection was 4.7 months.

None of the participants had treatment-emergent systemic adverse events, while subconjunctival hemorrhages commonly occurred as side-effects of the intravitreal injection procedure. All conjunctival hemorrhages resolved on their own. One patient reported floaters and a subjective decrease in quality of vision one month after the second injection, without a measurable decrease in vision. The subjective symptoms subsided within four weeks. None of the eyes showed an intraocular inflammatory reaction such as vitritis or retinal vasculitis during the postoperative period. There was no case of endophthalmitis or development of a choroidal neovascularization. In particular, there were no abnormalities detected in the region of the external eye including the cornea and conjunctiva, except for the hyposphagmata mentioned above. The IOP as assessed about 15 minutes after the injections was less than 25 mm Hg.

BCVA remained unchanged (*P*=0.08) during the study period with a mean BCVA of 1.62 ± 0.47 logMAR at baseline and of 1.28 ± 0.59 logMAR at study end (median: 1.10; range: 0.10 to 2.30). In a similar manner, IOP at baseline and IOP at study end did not differ significantly (13.8 ± 2.4 mm Hg versus 14.3 ± 2.6 mm Hg; *P*=0.20) (Table 1). None of the eyes showed an IOP higher than 21 mm Hg during the study period.

Nine patients of the 11 patients had a follow-up of at least 3 months (mean: 6.7 ± 2.7 months; median: 6.6 months; range: 3.1-11.9 months). The 9 patients received the panitumumab injections in doses of 0.6 mg (3 eyes; 3x2 Injections), 1.2mg (4 eyes; 1x1 injection, 2x2 injections; 1x3 injections) and 1.8 mg (2 eyes; 2x2 injections), respectively (Table 1).

Including only the 9 patients with a minimal follow-up of 3 months into the analysis, BCVA remained unchanged (*P*=0.15) during the study period with a mean BCVA of 1.65 ± 0.51 logMAR at baseline (median: 1.52; range: 1.00 to 2.30) and of 1.31 ± 0.65 at study end (median: 1.10; range: 0.10 to 2.30). In a similar manner, IOP at baseline and IOP at study end did not differ significantly (13.9 ± 2.6 mm Hg versus 14.3 ± 2.8 mm Hg; *P*=0.37) (Table 1). In a similar manner, the amplitude of the b-wave in the electrography did not differ significantly between baseline and study end.

In these 9 eyes with a follow-up >3 months, axial length measured at baseline did not differ significantly from axial length measured at study end (30.73 ± 1.03 mm versus 30.77 ± 1.19 mm; *P*=0.56). The mean non-significant change in axial length during the study period was 0.04 ± 0.21 mm (median: 0.07 mm; range: -0.33 to +0.30 mm) (Table 1). In univariate analysis, the change in axial length increased with longer axial length at baseline (standardized regression coefficient beta: 0.70; *P*=0.04) and younger age (beta: -0.68; *P*=0.04), while it was statistically independent of the follow-up duration (beta: 0.34; *P*=0.37). In a multivariable analysis, the change in axial length was associated neither with axial length at baseline (*P*=0.20), age (*P*=0.12) or follow-up duration (*P*=0.25).

The amplitude of the b-wave of the ERG did not differ significantly between the measurements obtained at baseline and at study end (P=0.59).

A roughly similar ratio is obtained when the systemically routinely applied dose of 420 mg panitumumab is compared with an intravitreally applied dose of 0.6 mg panitumumab.

Many modifications and other embodiments of the invention set forth herein will come to mind to the one skilled in the art to which the invention pertains having the benefit of the teachings presented in the foregoing description and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

### Example 3 - Influence of EGF and EGFR blocker on proliferation of RPE cells in vitro

Human RPE cell lines (ARPE-19 cells) were treated with different doses of EGF and EGF antibody as shown in Figures 5 and 6. Cell proliferation was examined by Cell Counting Kit-8 (CCK-8) and cell migration was examined by Scratch Assay as described in Dong et al. 2020.

EGF increased the proliferation of RPE cells in a dose-dependent manner, while the EGF antibody decreased the proliferation of RPE cells in a dose-dependent manner (all P<0.001) (Fig. 5). Correspondingly, EGF was associated with an increase in RPE cell migration in a dose-dependent manner, while the EGF antibody inhibited the RPE cell migration (all *P*<0.001) (Fig. 6)

### REFERENCES

Ahsan A, Ramanand SG, Bergin IL, Zhao L, Whitehead CE, Rehemtulla A, Ray D, Pratt WB, Lawrence TS, Nyati MK. Efficacy of an EGFR-specific peptide against EGFR-dependent cancer cell lines and tumor xenografts. Neoplasia. 2014 Feb;16(2):105-14.
Bikbov, M.M., Khalimov, T.A., Cerrada-Gimenez, M., Ragauskas, S., Kalesnykas, G. & Jonas, J.B. Compatibility of intravitreally applied epidermal growth factor and amphiregulin. Int. Ophthalmol. 41, 2053-2063 (2021).
Bikbov MM, Kazakbaeva GM, Panda-Jonas S, Khakimov DA, Gilemzianova LI, Miniazeva LA, Tuliakova AM, Fakhretdinova AA, Kazakbaev RA, Nuriev IF, Jonas JB. Safety and tolerability of intravitreal cetuximab in young and adult rabbits. Sci Rep. 2022 Jul 6;12(1):11454.
Bourne RR, Stevens GA, White RA, Smith JL, Flaxman SR, Price H, Jonas JB, Keeffe J, Leasher J, Naidoo K, Pesudovs K, Resnikoff S, Taylor HR; on behalf of the Vision Loss Expert Group. Causes of vision loss worldwide, 1990-2010: a systematic analysis. Lancet Glob Health. 2013 Dec;1(6):e339-e349.
Bublil EM, Yarden Y (April 2007). "The EGF receptor family: spearheading a merger of signaling and therapeutics". Current Opinion in Cell Biology. 19 (2): 124-34.
Balaratnasingam C, Messinger JD, Sloan KR, Yannuzzi LA, Freund KB, Curcio CA. Histologic and optical coherence tomographic correlations in drusenoid pigment epithelium detachment in age-related macular degeneration. Ophthalmology. 2017;124:644-656.
Cao D, Leong B, Messinger JD, Kar D, Ach T, Yannuzzi LA, Freund KB, Curcio CA. Hyperreflective Foci, Optical Coherence Tomography Progression Indicators in Age-Related Macular Degeneration, Include Transdifferentiated Retinal Pigment Epithelium. Invest Ophthalmol Vis Sci. 2021 Aug 2;62(10):34.
Chen L, Cao D, Messinger JD, Ach T, Ferrara D, Freund KB, Curcio CA. Histology and clinical imaging lifecycle of black pigment in fibrosis secondary to neovascular age-related macular degeneration. Exp Eye Res. 2022 Jan;214:108882.
Curcio CA, Zanzottera EC, Ach T, Balaratnasingam C, Freund KB. Activated retinal pigment epithelium, an optical coherence tomography biomarker for progression in age-related macular degeneration. Invest Ophthalmol Vis Sci. 2017;58: BIO211-BIO226.
Cunningham D, Humblet Y, Siena S, Khayat D, Bleiberg H, Santoro A, Bets D, Mueser M, Harstrick A, Verslype C, Chau I, Van Cutsem E. Cetuximab monotherapy and cetuximab plus irinotecan in irinotecan-refractory metastatic colorectal cancer. N Engl J Med. 2004 Jul 22;351(4):337-45.
Dong L, Shi XH, Kang YK, et al. Amphiregulin and ocular axial length. Acta Ophthalmol 2019;97:e460-70.
Dong L, Shi XH, Li YF, et al. Blockade of epidermal growth factor and its receptor and axial elongation in experimental myopia. FASEB J 2020 ;34 :13654-70.
Dong L, Zhang RH, Zhou WD, et al. Epiregulin, epigen and betacellulin antibodies and axial elongation in young guinea pigs with lens-induced myopization. BMC Ophthalmol 2022;22:193.
Douillard JY, Oliner KS, Siena S, et al. Panitumumab-FOLFOX4 treatment and RAS mutations in colorectal cancer. N Engl J Med 2013;369:1023-34.
Ferguson KM. Structure-based view of epidermal growth factor receptor regulation. Annu Rev Biophys. 2008;37:353-73.
Flaxman SR, Bourne RRA, Resnikoff S, Ackland P, Braithwaite T, Cicinelli MV, Das A, Jonas JB, Keeffe J, Kempen JH, LeasherJ, Limburg H, Naidoo K, Pesudovs K, Silvester A, Stevens GA, Tahhan N, Wong TY, Taylor HR; Vision Loss Expert Group of the Global Burden of Disease Study. Global causes of blindness and distance vision impairment 1990-2020: a systematic review and meta-analysis. Lancet Glob Health. 2017;5:e1221-e1234.
Fleckenstein M, Mitchell P, Freund KB, Sadda S, Holz FG, Brittain C, Henry EC, Ferrara D. The Progression of Geographic Atrophy Secondary to Age-Related Macular Degeneration. Ophthalmology. 2018 Mar;125(3):369-390.
Guymer RH, Rosenfeld PJ, Curcio CA, et al. Incomplete retinal pigment epithelial and outer retinal atrophy (iRORA) in age-related macular degeneration: CAM Report 4. Ophthalmology. 2020;127:394-409.
Guymer RH, Campbell TG. Age-related macular degeneration. Lancet. 2023 Apr 29;401(10386): 1459-1472
Hecht JR, Patnaik A, Berlin J, Venook A, Malik I, Tchekmedyian S, Navale L, Amado RG, Meropol NJ. Panitumumab monotherapy in patients with previously treated metastatic colorectal cancer. Cancer. 2007 Sep 1;110(5):980-8.
Hurwitz H, Fehrenbacher L, Novotny W, et al. Bevacizumab plus irinotecan, fluorouracil, and leucovorin for metastatic colorectal cancer. N Engl J Med 2004;350:2335-42.
Im YH, Im SA, Semiglazov V, Ciruelos E, Schneeweiss A, Loi S, Monturus E, Clark E, Knott A, Restuccia E, Benyunes MC, Cortés J; CLEOPATRA study group. Pertuzumab, trastuzumab, and docetaxel for HER2-positive metastatic breast cancer (CLEOPATRA): end-of-study results from a double-blind, randomised, placebo-controlled, phase 3 study. Lancet Oncol. 2020 Apr;21(4):519-530.
Jiang WJ, Song HX, Li SY, et al. Amphiregulin antibody and reduction of axial elongation in experimental myopia. EBioMedicine 2017;17:134-44.
Jonas SB, Panda-Jonas S, Jonas JB, Jonas RA. Histology of neovascular myopic macular degeneration. Sci Rep. 2021;11(1):21908.
Khaliq, A., Jarvis-Evans, J., McLeod, D., Boulton, M., 1996. Oxygen modulates the response of the retinal pigment epithelium to basic fibroblast growth factor and epidermal growth factor by receptor regulation. Invest. Ophthalmol. Vis. Sci. 37:436-443.
Lim LS, Mitchell P, Seddon JM, Holz FG, Wong TY. Age-related macular degeneration. Lancet. 2012 May 5;379(9827):1728-38.
Martin DF, Maguire MG, Ying GS, Grunwald JE, Fine SL, Jaffe GJ. Ranibizumab and bevacizumab for neovascular age-related macular degeneration. N Engl J Med. 2011 May 19;364(20):1897-908.
Mitchell P, Liew G, Gopinath B, Wong TY. Age-related macular degeneration. Lancet. 2018 Sep 29;392(10153):1147-1159.
Ohno-Matsui K, Kawasaki R, Jonas JB, et al. International photographic classification and grading system for myopic maculopathy. Am J Ophthalmol 2015;159:877-83.
Piccart-Gebhart MJ, Procter M, Leyland-Jones B, Goldhirsch A, Untch M, Smith I, Gianni L, Baselga J, Bell R, Jackisch C, Cameron D, Dowsett M, Barrios CH, Steger G, Huang CS, Andersson M, Inbar M, Lichinitser M, Láng I, Nitz U, Iwata H, Thomssen C, Lohrisch C, Suter TM, Rüschoff J, Suto T, Greatorex V, Ward C, Straehle C, McFadden E, Dolci MS, Gelber RD; Herceptin Adjuvant (HERA) Trial Study Team. Trastuzumab after adjuvant chemotherapy in HER2-positive breast cancer. N Engl J Med. 2005 Oct 20;353(16):1659-72.
Ramakrishnan MS, Eswaraiah A, Crombet T, Piedra P, Saurez G, Iyer H, Arvind AS. Nimotuzumab, a promising therapeutic monoclonal for treatment of tumors of epithelial origin. MAbs. 2009 Jan-Feb;1(1):41-8.
Rosenfeld PJ, Brown DM, Heier JS, Boyer DS, Kaiser PK, Chung CY, Kim RY; MARINA Study Group. Ranibizumab for neovascular age-related macular degeneration. N Engl J Med. 2006 Oct 5;355(14):1419-31.
Sadda SR, Guymer R, Holz FG, Schmitz-Valckenberg S, Curcio CA, Bird AC, Blodi BA, Bottoni F, Chakravarthy U, Chew EY, Csaky K, Danis RP, Fleckenstein M, Freund KB, Grunwald J, Hoyng CB, Jaffe GJ, Liakopoulos S, Monés JM, Pauleikhoff D, Rosenfeld PJ, Sarraf D, Spaide RF, Tadayoni R, Tufail A, Wolf S, Staurenghi G. Consensus Definition for Atrophy Associated with Age-Related Macular Degeneration on OCT: Classification of Atrophy Report 3. Ophthalmology. 2018 Apr;125(4):537-548.
Spaide RF, Jaffe GJ, Sarraf D, Freund KB, Sadda SR, Staurenghi G, Waheed NK, Chakravarthy U, Rosenfeld PJ, Holz FG, Souied EH, Cohen SY, Querques G, Ohno-Matsui K, Boyer D, Gaudric A, Blodi B, Baumal CR, Li X, Coscas GJ, Brucker A, Singerman L, Luthert P, Schmitz-Valckenberg S, Schmidt-Erfurth U, Grossniklaus HE, Wilson DJ, Guymer R, Yannuzzi LA, Chew EY, Csaky K, Monés JM, Pauleikhoff D, Tadayoni R, Fujimoto J. Consensus Nomenclature for Reporting Neovascular Age-Related Macular Degeneration Data: Consensus on Neovascular Age-Related Macular Degeneration Nomenclature Study Group. Ophthalmology. 2020 May;127(5):616-636.
Schaub F, Schiller P, Hoerster R, Kraus D, Holz FG, Guthoff R, Agostini H, Spitzer MS, Wiedemann P, Lommatzsch A, Boden KT, Dimopoulos S, Bemme S, Tamm S, Maier M, RoiderJ, Enders P, Altay L, Fauser S, Kirchhof B;. Intravitreal 5-Fluorouracil and Heparin to Prevent Proliferative Vitreoretinopathy: Results from a Randomized Clinical Trial. Ophthalmology. 2022 Oct;129(10):1129-1141.
Schlessinger J. Ligand-induced, receptor-mediated dimerization and activation of EGF receptor. Cell 2002;110:669-72.
Vanhoefer U, Tewes M, Rojo F, Dirsch O, Schleucher N, Rosen O, Tillner J, Kovar A, Braun AH, Trarbach T, Seeber S, Harstrick A, Baselga J. Phase I study of the humanized antiepidermal growth factor receptor monoclonal antibody EMD72000 in patients with advanced solid tumors that express the epidermal growth factor receptor. J Clin Oncol. 2004 Jan 1 ;22(1):175-84.
Zanzottera EC, Messinger JD, Ach T, Smith RT, Curcio CA. Subducted and melanotic cells in advanced age-related macular degeneration are derived from retinal pigment epithelium. Invest Ophthalmol Vis Sci. 2015;56:3269-3278.

## Claims

1. A pharmaceutical composition for use in the treatment of a disease involving unwanted migration, proliferation and metaplasia of retinal pigment epithelium (RPE) cells in a subject, comprising at least one epidermal growth factor receptor (EGFR) antagonist.

2. The pharmaceutical composition for use according to claim 1, wherein the disease is selected from exudative age-related macular degeneration (AMD), intermediate stage AMD, macular neovascularization in pathologic myopia, polypoidal choroidal vasculopathy (PCV), any other disease located in the macular region and **characterized by** a neovascularization, originating in the choroid and extending into the sub-RPE compartment and/or into the subretinal space, and proliferative vitreoretinopathy (PVR).

3. According to one embodiment, the EGFR antagonist decreases the EGFR signaling by at least 10%, at least 20%, at least 30%, at least 40%, at least 50%, at least 60%, at least 70%, at least 75%, at least 80%, at least 85%, at least 90%, at least 92.5%, at least 95%, at least 97%, at least 98%, at least 98,5%, at least 99%, at least 99.25%, at least 99.5%, or at least 99.75% as compared to normal physiologic levels.

4. The pharmaceutical composition for use according to claim 2, wherein said EGFR antagonist is selected from the group consisting of:
(a) an antibody-based molecule, capable of binding to EGFR and decreasing EGFR signaling;
(b) an antibody-based molecule, capable of binding to an EGF family member and decreasing EGFR signaling;
(c) a small molecule EGFR tyrosine kinase inhibitor;
(d) a peptide inhibitor that is capable of binding to EGFR and decreasing EGFR signaling;
(e) a small interfering RNA (siRNA) agent capable of reducing the expression of EGFR;
(f) a CRISPR/Cas9 construct capable of knocking out the EGFR gene, which will directly address the EGFR; and
(g) an mRNA molecule encoding an antibody - based molecule capable of binding to EGFR and decreasing EGFR signaling or encoding a peptide inhibitor that is capable of binding to EGFR and decreasing EGFR signaling.

5. The pharmaceutical composition for use according to any of the preceding claims, wherein the antibody-based molecule is selected from an antibody-based molecule blocking the binding of the EGF family members to EGFR, an antibody-based molecule capable of binding to an EGF family member and decreasing EGFR signaling; an antibody-based molecule inhibiting the dimerization of EGFR, or an antibody-based molecule inhibiting the tyrosine kinase activity of EGFR.

6. The pharmaceutical composition for use according to any of the preceding claims, wherein the antibody-based molecule is selected from an antibody, an antibody fragment, an antibody mimetic.

7. The pharmaceutical composition for use according to claim 6, wherein the antibody is preferably selected from panitumumab, cetuximab, nimotuzumab, matuzumab, pertuzumab and trastuzumab.

8. The pharmaceutical composition for use according to claim 6, wherein said antibody fragment is selected from the group consisting of Fab fragments, F(ab')₂ fragments and Fab' fragments and/or the antibody mimetic is selected from the group consisting of single-chain variable fragments (scFv), single-domain antibodies, affibodies, affilins, affimers, affitins, anticalins, DARPins, monobodies, and peptide aptamers.

9. The pharmaceutical composition for use according to any one of claims 1 to 4, wherein the small molecule EGFR tyrosine kinase inhibitor is selected from gefitinib, erlotinib, lapatinib, icotinib, afatinib, neratinib, dacomitinib, almonertinib, olmutinib, and osimertinib, brigatinib, vandetanib, and pyrotinib.

10. The pharmaceutical composition for use according to any one of claims 1 to 4, wherein the peptide inhibitor is selected from a peptide inhibiting the binding of the EGF family members to EGFR, a peptide inhibiting the dimerization of EGFR, or a peptide inhibiting the tyrosine kinase activity of EGFR and wherein the peptide inhibitor has a length of at most 80 amino acids, preferably at most 60 amino acids, more preferably 40 amino acids, most preferably, 20 amino acids.

11. The pharmaceutical composition for use according to any one of claims 1 to 4, wherein the antisense strand of such siRNA agents, i.e., the strand targeting EGFR mRNA, contains a stretch of at least 50 consecutive nucleic acids of one of the nucleic acid sequence with the SEQ ID NO: 1.

12. The pharmaceutical composition for use according to any one of claims 1 to 4, the mRNA molecule encodes an antibody selected from panitumumab, cetuximab, nimotuzumab, matuzumab, pertuzumab and trastuzumab or a peptide inhibitor according to claim 7.

13. The pharmaceutical composition for use according to any one of the preceding claims, wherein said use comprises administering the pharmaceutical composition intravitreally, preferably in the temporal inferior quadrant of the eye more preferably through the conjunctiva, sclera and pars plana into the vitreous cavity.

14. The pharmaceutical composition for use according to any one of the preceding claims, wherein said use comprises administering the pharmaceutical composition in a distance of 1 mm to 6 mm from the limbus, more preferably in a distance of 2 mm to 5 mm, most preferably in a distance of 3 mm to 4 mm posterior to the corneal limbus.

15. The pharmaceutical composition for use according to any one of claims 5 to 8, wherein said use comprises administering the pharmaceutical composition with the antibody-based molecule in a total dosage in the range of 0.1 mg to 6 mg per eye, preferably in the range of 0.30 µg to 3 mg, more preferably in the range of 0.5 mg to 2 mg.
